# EUROPÄISCHE PATENTANMELDUNG

(11) **EP 1 072 258 A1**
(43) Veröffentlichungstag der Anmeldung: **31.01.2001**
(21) Anmeldenummer: 99810682.7
(22) Anmeldetag: 28.07.1999
(51) Int. Cl.: A61K 9/48, A61K 35/74

(54) **Eine Kapsel für die Freisetzung von Bakterien, enthaltend ein Bakterienlyophilisat und ein Verfahren zu ihrer Herstellung**

(71) Anmelder: Greither, Peter, CH-9533 Kirchberg (CH)
(72) Erfinder: Engel, Dieter, 9524 Zuzwil (CH); Brocker, Erich, Dr., 9533 Kirchberg (CH)
(74) Vertreter: Müller, Christoph Emanuel

(57) **Zusammenfassung**

Eine Kapsel für die Freisetzung von Bakterien, enthaltend ein Bakterienlyophilisat, vorzugsweise lyophilisierte Milchsäurebakterien, und Füllstoffe, wobei der Wassergehalt (im thermodynamischen Gleichgewicht) der Kapselhülle höchstens 7% (w/w), vorzugsweise weniger als 3% (w/w) beträgt. Vorteil ist, bei gegebenem Wassergehalt von 4-10% des mit den lyophilisierten Bakterien vermischten Füllguts, eine verbesserte Überlebensrate der Bakterien nach Lagerung.

## Beschreibung

Die Erfindung betrifft eine Kapsel für die pharmazeutische Dosierung und für die Nahrungsergänzung enthaltend ein Bakterienlyophilisat und Füllstoffe sowie ein Verfahren zu ihrer Herstellung gemäss dem Oberbegriff der unabhängigen Patentansprüche.

Die bakterielle Mikroflora des menschlichen Darms oder anderer Körperhohlräume ist ein regulärer Bestandteil der Körperfunktionen. Darmbakterien helfen bei der Verdauung der Nahrung, stellen als einzige Quelle lebenswichtige Vitamine her (z.B. B12) und behindern durch Wachstumskompetition oder bakterizide Substanzen die Besiedlung mit pathogenen Mikroorganismen. Die Behandlung oder Prävention von Störungen der mikrobiellen Flora erfolgt seit langem durch direkte Aufnahme von lebenden Bakterien wie z.B. Lactobacillusstämmen. Dies kann z. B. durch Aufnahme von fermentierten, frischen Milchprodukten erfolgen. Derartige als Nahrungssupplement gehandhabte Mikroorganismenkulturen werden als probiotische Organismen bezeichnet (Probiotics - The scientific basis, ed. R. Fuller, Chapman and Hall 1992). Ausgewählte probiotische Stämme können immunmodulatorische Funktion haben. Die Einnahme probiotischer Bakterien ist ein Therapiemittel bei der Behandlung akuter Störung des Darmtraktes wie z.B. bei Durchfallerkrankungen oder anderer Erkrankungen (Feeding from Bifidobacterium bifidum and Streptococcus thermophilus to infants in hospital for prevention of diarroea and shedding rotavirus, The Lancet 344, J. M. Saavedra et al., 1994; und: Factors to consider when selecting a culture of Lactobacillus acidophilus as a dietary adjunct to produce a hypocholesterolemic effect in öhumans, J. Dairy Sci. 73, S.E. Gilliland et al. 1990).

Eine Aufnahme von Lactobacilli ganz bestimmter, selektionierter Stämme ist in der Regel nicht durch den Verzehr von Lebensmittelfrischprodukten mit Lactobazillen möglich, da enge Grenzen durch die Gesetzgebung, die Kultur-Matrix (z.B. Yoghurt), Geschmack, Überlebensrate bedingt durch Aufbewahrungszeit und -temperatur gegeben sind. In normalen Nahrungsmitteln ist die Lebensdauer dieser erwünschten Bakterien in den gewünschten hohen Populationen auf höchstens einige Tage beschränkt. Der gezielte Einsatz probiotischer Organismen erfordert die Aufnahme einer gewissen Mindestdosis (ca. 10⁸ Bakterien) von noch lebensfähigen Mikroorganismen, damit diese trotz herrschenden Wachstumskompetition einen ausreichenden Effekten erzielen können. Gerade auch spezielle Anwendungen in der Medizin (z.B. als Vaginalsubsitorium) erfordern lagerfähige, kompakte Darreichungsformen, in der die Bakterien in einer lebensfähigen Form konserviert sind.
Kälte und Trockenheit sind die zwei Grundprinzipien der Konservierung lebender Mikroorganismen. Viele Bakterienstämme können durch Gefriertrocknung, auch als Lyophilisierung bezeichnet, als Pulver für eine begrenzte Zeit lebensfähig gelagert werden.

US 5 501 857 beschreibt eine Steckkapsel aus Hartgelatine. Sie enthält in einer inneren, zweiten Steckkapsel ein Bakterienlyophilisat. Nach sechs Monaten Lagerung bei Raumtemperatur ist die Überlebensrate auf 53% gesunken.
Die unbefriedigende Überlebensrate ist ein Grundproblem der bekannten Gelatinekapseln für probiotische Bakterien. I. A. müssen die bisher bekannten Kapseln daher daher kühl, bei ca. 4°C, gelagert werden.
JP 61151127 beschreibt analog eine Weichgelatinekapsel mit lyophilisierten Bifidusbakterien. Zur Verbesserung der Überlebensrate der Bakterien nach oraler Einnahme ist die Kapsel mit einem magensaftresistenten Überzug versehen.
Die Lagerstabilität wird durch diese Massnahme aber nicht verbessert.

WO 97/29762 beschreibt die Mikroverkapselung von Lactobacillus oder Bifidobakterium. Nach Entfernen und Auswaschen des Wachstumsmediums werden die Bakterien zunächst lyophilisiert, dann in einem Gel eingeschlossen und noch einmal gefriergetrocknet. Als eine mögliche Darreichungsform werden Kapseln aus Hart- oder Weichgelatine mit einem gewissen Wassergehalt genannt.
Die Mikroverkapselung ist eine aufwendige Methode; das Quellen der Verkapselungsmatrix nach Auflösung der Kapsel behindert den Zugang der darin eingeschlossenen probiotischen Mikroorganismen zu den Schleimhäuten, zwecks Adhäsion der Bakterien am Epithel.

Allen diesen aus dem Stand der Technik bekannten Ausführungen ist gemeinsam, dass eine Kapsel aufgrund der geringen mechanischen Belastbarkeit von Bakterienlyophilisaten die geeignetste unter den möglichen Darreichungsformen ist. Als Steckkapseln oder als nach dem Rotary-Die-Verfahren hergestellte Weichkapseln finden dabei stets Gelatinekapseln Verwendung.

Aufgabe der vorliegenden Erfindung ist es die Nachteile des Bekannten zu vermeiden und eine einfach herzustellende Darreichungsform für lyophilisierte, probiotische Mikroorganismen zu beschreiben, die die Überlebensrate der Bakterien während der Lagerung verbessert.

Diese Aufgabe wird mit einer Kapsel gelöst, welche die kennzeichnenden Merkmale der unabhängigen Patentansprüche 1 und 10 aufweist. Ein weiterer Gegenstand der vorliegenden Erfindung ist ein Verfahren zur Herstellung einer solchen Kapsel gemäss den Merkmalen des unabhängigen Patentanspruchs 12.

Eine Kapsel mit einem geringeren Wassergehalt der Kapselhülle erhöht die Lagerstabilität der darin zusammen mit Füllstoffen enthaltenen Bakterien wesentlich.

Eine Kapsel für die pharmazeutische Dosierung und für die Nahrungsergänzung enthält in einer Kapselhülle mindestens ein Bakterienlyophilisat, vorzugsweise lyophilisierte Milchsäurebakterien, und mindestens einen Füllstoff, wobei der Wassergehalt der Kapselhülle 2-7% (w/w), vorzugsweise weniger als 3% (w/w) beträgt. Der Vorteil dieser Ausführung ist, dass der Wassergehalt der Kapselhülle sich damit höchstens im Bereich des üblichen Wassergehalts der Füllstoffe bewegt. Der Wassergehalt der Füllstoffe bei herkömmlichen Darreichungsformen liegt in der Grössenordnung 4 bis 10% (w/w). Der Wassergehalt herkömmlicher Gelatinekapseln liegt zwischen 10-15% (w/w).
Als Wassergehalt im Sinne der vorliegenden Erfindung gilt der Gewichtsverlust nach mindestens 24-stündiger Trocknung einer Probe bei 60°C über Phosphorpentoxid bis zur Gewichtskonstanz. Es handelt sich um den Wassergehalt im thermodynamischen Gleichgewicht mit der umgebenden Gasphase (,Equilibrium Moisture Content', *EMC,* vgl.: Die Kapsel, Grundlagen, Technologie und Biopharmazie einer modernen Arzneiform, ed. Fahrig, W. et al., Wissenschaftliche Verlagsgesellschaft mbH Stuttgart 1983 und The Theory and Practice of Industrial Pharmacy, Lachman, L. et al., Lea/Febiger, Philadelphia und Aqueous Polymeric Coatings for Pharmaceutical Dosage Forms, ed. McGinity, J., Marcel Dekker Inc. 1997). Im Gleichgewicht besteht keine treibende Kraft zum Massentransfer von Wasser gegenüber einer Gasphase; der Dampfdruck des Materials entspricht dem in der Gasphase. Das in der Kapselhülle enthaltene Wasser ist aber vermittels der Gasphase den lyophilisierten Bakterien zugänglich, deren effektiver Wassergehalt nach der Trocknung im Vakuum gering ist. Ein zu hoher Wassergehalt der Kapselhülle kann daher zur latenten Aktivierung der getrockneten, lyophilisierten Bakterien führen und beeinträchtigt die Überlebensrate während der Lagerung.

Es wird zudem verhindert, dass die als Mischung mit dem Bakterienlyophilisat vorliegenden Füllstoffe in synergistischer Weise Wasser aus der Kapselhülle in das Innere der Kapsel ziehen und so unmittelbar den getrockneten Bakterien zuführen. Die geringe Sorption von Wasser aus der Kapselhülle bedingt auch, dass die mechanischen Eigenschaften der Kapselhülle während der Lagerung stabil bleiben. Wasserentzug führt bei Kapselmaterialien mit guten Eigenschaften bei höheren Wassergehalten (z.B. Gelatinekapseln) zu Versprödung; derartige mit einem Füllgut sehr geringen Wassergehalts befüllte Kapseln können daher nicht strikt trocken unter Luftabschluss gelagert werden.
Der *EMC*-Wert ist abhängig von Temperatur und relativer Feuchtigkeit (rH) der ein Material umgebenden Gasphase (Lachman, ibd.and McGinity, ibd., S.184). Mit zunehmender Feuchtigkeit (rH) der Gasphase nimmt auch der Wassergehalt des Materials zu (Sorption), wobei sowohl die Aufnahmerate bis zum Erreichen des Gleichgewichts wie der Anstieg des Wassergehaltes im Gleichgewicht (Sorption) materialspezifisch sind. Der Anstieg erfolgt i.A. exponentiell, wobei die Steigung stark materialabhängig ist. Das Erreichen eines Wassergehalts des Kapselmaterials im Sinne dieser Erfindung setzt also, bei gegebener Luftfeuchtigkeit, die Verwendung eines in Bezug auf seinen *EMC* geeigneten Kapselmaterials voraus, d.h. eines Materials niedriger Sorption, sowie die Verarbeitung und Lagerung der Kapseln bei geeigneter, konstanter Luftfeuchtigkeit. Beispielsweise erlauben herkömmliche Gelatinekapseln auch bei Einsatz von nichtwässrigen Weichmachern nicht das Erreichen eines *EMC* von weniger als 7%, oder sogar weniger als 3%.

Ein Kapselmaterial im Sinne der vorliegenden Erfindung zeichnet sich durch eine geringe Sorption und damit über einen weiten Bereich der Luftfeuchtigkeit niedrigen *EMC* aus. Ein Material im Sinne der vorliegenden Erfindung wird bei einer relativen Luftfeuchtigkeit (rH) von höchstens 55% einen Wassergehalt *(EMC)* von höchstens 7% aufweisen.

Geeignete Kapselmaterialien sind Zellulosederivate (z.B. Hydroxypropylcellulose, Cellulosea-cetate-phtalate, Ethylcellulose; vgl. Aqueous Polymeric Coatings for Pharmaceutical Dosage Forms, ed. McGinity, J., Marcel Dekker Inc. 1997, S.184) bzw. Polysacharidderivate, z.B. Xanthan oder Agar-Agar. Es ist aber auch jedes andere Material verwendbar, das einen geringen Wassergehalt und geeignete Löslichkeitseigenschaften besitzt, z.B. Schellack oder ein synthetisches Material wie z.B. Polyvinylalkohol (PVA).
In einer bevorzugten Ausführungsform besteht die Kapselhülle aus einem Zellulosederivat, vorzugsweise Hydroxypropylmethyl-Cellulose (HPMC). HPMC verbindet in idealer Weise die genannten Eigenschaften von geringem Wassergehalt, guten mechanischen Eigenschaften und guten Löslichkeitseigenschaften in wässrigem Medium in einer für die Darreichungsform als Kapsel geeigneten Zusammensetzung. Dies gilt auch für andere Alkoxy- bzw. Hydroxy-alkoxy-Derivate der Cellulose.
HPMC ist ein in der Galenik seit langem bekanntes Material (McGinity, ibd.) und wird von verschiedenen Herstellern angeboten. Das Molekulargewicht von verschiedenen HPMC Rohstoffen liegt in der Grössenordnung von ca. 10.000-120.000. Kapselhüllen haben einen Anteil von ca. mehr als 70% (w/w) HPMC. Der Substitutionsgrad (vgl. die US- oder Europäische Pharmacopeia) bestimmt die Löslichkeitseigenschaften und den Wassergehalt des Kapselmaterials. Bei üblichen HPMC Materialien liegt der Substitutionsgrad im Bereich von 15-30% (Methoxy) und 3-35% (Hydroxypropoxy); es sind beliebige Kombinationen beider Substitutionsbereiche möglich. Die (Hydroxy-)Alkoxygruppen stören die besonders starre Cellulosestruktur und erlauben das Optimieren der Materialeigenschaften. Es ist aber auch möglich, andere als die oben genannten Cellulosederivate zu verwenden, z.B. HPMC-acetat-succinat (HPMCAS) mit einem Wassergehalt (und Sorptionsverhalten) im Sinne der vorliegenden Erfindung. HPMCAS weist grössere Magensaftresistenz auf; ein pH >5 ist zur Auflösung erforderlich. Es sind aber auch andere Cellulosederivate denkbar, wie z.B. Hydroxyethyl-, Hydroxypropyl-, Hydroxyethylmethyl-, Methylpropyl- oder Ethyl-Cellulosen in geeigneten Substitutionsgraden.
Übliche Hartkapseln aus HPMC besitzen einen Wassergehalt (*EMC*)um die 3% (w/w); die Aufnahme von Wasser bei einer relativen Luftfeuchtigkeit von 11 bis 93% resultiert in einem Wassergehalt von 2,1 bis 22,6% (w/w) (Aequeous Polymeric Coatings for pharmaceutical dosage forms, ed. J.W. McGinity, Marcel Dekker Inc., N.Y. 1997). Dabei übersteigt der Wassergehalt aber bis zu einer Luftfeuchtigkeit von 53% nicht die Schwelle von 6% (w/w). Zudem ist die Aufnahmerate von Wasser aus der Luft ein langsamer Prozess, im Vergleich z.B. zu Hartgelatinekapseln. Die mechanischen Eigenschaften derartiger Kapseln aus HPMC sind denen herkömmlicher Kapselmaterialien ebenbürtig. Eine mögliche Ausführungsform ist z.B. in US 5 264 223 beschrieben.

In einer weiteren, bevorzugten Ausführungsform besteht die Kapselhülle aus Stärke, vorzugsweise einem Stärkederivat. Bei derartigen Derivaten kann es sich um veresterte oder veretherte Stärkederivate handeln. Derartige Kapselhüllen haben ebenfalls die oben genannten, vorteilhaften Eigenschaften.

Bei dem Bakterienlyophilisat handelt es sich um probiotische, gefriergetrocknete Bakterien (Probiotics - The scientific basis, ed. R. Fuller, Chapman and Hall 1992). Es kann sich aber auch um den Bakterien bzgl. Wachstumsverhalten und probiotischer Eigenschaften vergleichbare, durch Gefriertrocknung konservierbare einzellige eukaryontische Mikroorganismen, nämlich Hefen, handeln. Die Gefriertrocknung ist ein in der Mikrobiologie seit langem bekanntes Konservierungsverfahren zur trocknen Lagerung von lebensfähigen Bakterien über Monate oder Jahre. Einzelne Stämme, insbesondere auch einzelne, selektionierte Varianten bekannter Stämme, können dabei wesentlich geringere Überlebensraten nach Gefriertrocknung aufweisen. Ein Standardverfahren ist, Bakterien aus Flüssigkultur unmittelbar nach Erreichen der mid-log oder stationären Phase von der Wachstumstemperatur auf ca. 4°C kühlen, das Wachstumsmedium auszuwaschen und die Bakterien in flüssigem Stickstoff schockzugefrieren (snap-freezing) und im Hochvakuum zu lyophilisieren. Es gibt aber auch modifizierte Verfahren. Bei dem Bakterienlyophylisat kann es sich um einen einzigen, es kann sich aber auch um eine Mischung verschiedener Bakterienstämme handeln. Vorzugsweise für Milchsäurebakterien ist diese Art der Konservierung in der Galenik geeignet; es handelt sich zwar um Gram-positive, aber nicht zur Bildung von Dauerformen (Sporen) befähigte Bakterien mit anaeroben Metabolismus (Gärung). Zudem sind einzelne Species wie Bifidobakterium obligat anaerob, während Lactobacillus zumindestens Sauerstoff-tolerant ist; die zuverlässige Konservierung gerade dieser Stämme im lebensfähigen, aber nicht aktiv metabolisierendem Zustand ist daher besonders wichtig.

Erfindungsgemässe Füllmaterialien können Mono- oder Oligosaccharide, mikrokristalline Cellulose (Avicel), Polyvinylpyrrolidon, Stearate oder Stärkederivate sein. Es sind aber auch andere Füllstoffe möglich, wie z.B. spezielle Pflanzenextrakte mit spezifisch immunmodulierender Wirkung oder selektiv bakterizider Wirkung. Es ist auch möglich, spezielle Oligosacharide mit anti-adhesiver Wirkung gegen Pathogene (wie sie z.B. auch in Preiselbeersaft vorkommen) als Füllstoffkomponente zuzufügen. Es ist auch möglich, eine Kapsel im Sinne der vorliegenden Erfindung als Doppelkapsel (Kapsel in einer Kapsel) auszulegen, wie aus dem Stand der Technik bekannt.

In einer weiteren bevorzugten Ausführungsform der erfindungsgemässen Kapsel umfassen die Füllstoffe eine oder mehrere das Wachstum von Bakterien fördernden Substanzen, vorzugsweise Lactose oder Oligofruktose oder eine Kombination davon. Es kann sich aber auch um Lactulose, Glucose, Glucosephosphat, andere Mono-, Oligo- oder Polysaccharide wie Glykogen, Stärkederivate oder Maltodextrin handeln. Es kann sich um eine beliebige Mischung solcher wachstumsfördernder Stoffe handeln. Sowohl zur Erhaltung der Lebensfähigkeit bei latentem Wachstum nach Aktivierung der Bakterien während der Lagerung bzw. beim Auflösen der Kapseln sichern diese Füllstoffe den Energiestoffwechsel der Bakterien. Die Darmflora ist das natürliche Habitat von Milchsäure-bildenden Bakterien, die sonst nur noch in Milch selbst vorkommen. Sie vermögen als einzige Bakterien Lactose zu vergären. Die Lactatausscheidung ist ein wichtiger Mechanismus, um durch einen niedrigen pH im extrazellulären Medium die Besiedlung der Darmflora durch Pathogene zu verhindern. Milchsäurebakterien wie Lactobacillus oder Bifidumbakterien sind deshalb die oft verwandete probiotischen Bakterien. Indem man als Füllmaterial Lactose beimischt, ist bei Auflösung der Kapsel im Darm unmittelbar eine Energiequelle zur Vermehrung und zur Wiederbelebung des Stoffwechsels im Zuge der Rehydratisierung vorhanden. Milchsäurebakterien verwenden auch Fruktooligosacharide als eine Energiequelle. Oligofructose (oder Fructose-Oligosaccharid) ist die Oligosaccharidfraktion des Inulin, einem pflanzlichem Extrakt, und ist bekannt. Das Polysacharid besteht aus Glucosylfructosedisaccharideinheiten mit einem mittleren Polymerisationsgrad von 10 Disacharideinheiten und maximal bis zu 60 Disaccharideinheiten. Im Sinne der vorliegenden Erfindung kann die Oligofructose auch einen geringen Anteil von Nicht-Glucose, Nicht-Fructose Komponenten enthalten, wie z.B. Polyalkohole, Sorbitol oder Mannitol. Vorzugsweise wird die als Füllmaterial verwendete Laktose beta-Laktose sein, da diese mit 62 g/l eine ungefähr 10 Mal höhere Löslichkeit im Wasser als das alpha-Anomere aufweist.

In einer bevorzugten Ausführungsform weist das Füllmaterial zum Zeitpunkt der Verkapselung einen Wassergehalt *(EMC)* von höchstens 2% (w/w), vorzugsweise von weniger als 1% (w/w) auf. Vorteil ist ein noch geringerer Wassergehalt in der unmittelbaren Umgebung der lyophilisierten, ungeschützten Bakterien. Bakterien im Sinne der vorliegenden Erfindung sind unmittelbar nach Auswaschen eines Kulturmediums durch Gefriertrocknung (Lyophilisierung) konserviert worden und verfügen über keine weitere, schützende Umhüllung wie z.B. Mikroverkapselung. Demzufolge spielt der Wassergehalt des unmittelbar in der Mischung die Bakterien umhüllenden Füllgutes eine wesentliche Rolle dabei, Feuchtigkeitszutritt zu den Bakterien zu verhindern. In Kombination mit einer erfindungsgemässen Kapselhülle mit geringer Sorption/mit niedrigem *EMC,* und Auswahl geeigneter Keime, ergibt sich, ausgehend von einer Keimzahl von mindestens 10⁸ Bakterien pro Kapsel, eine verbesserte Überlebensrate (bei einer Lagerung bei 20-25°C und 20-80% rH) gegenüber den bekannten Darreichungsformen. Die Trocknung des Füllmaterials auf einen Wassergehalt von weniger als 1% (w/w) erfordert die Lagerung und Handhabung des so getrockneten Materials in einer Atmosphäre mit höchstens 30%, vorzugsweise weniger als 10% rH.

In einer weiteren bevorzugten Ausführungsform umfasst das Bakterienlyophilisat einen oder mehrere magensaftresistente, vorzugsweise auch gallentolerante, Bakterienstämme. Die Keimzahl derartiger Stämme wird bei der Passage durch das saure Milieu des Magens und durch die Gallensäuren nur unwesentlich verringert. Es ist möglich, z.B. den Stamm Lactobacillus acidophyllus La-5 (Firma Chr. Hansen Inc./Illinois) oder den Stamm Bifidobacterium Bifidus Bb-12 (Firma Chr. Hansen Inc./Illinois) oder eine Mischung solcher Stämme zu verwenden. Im Kontext der vorliegenden Erfindung wird Magensaftresistenz dadurch erfüllt, dass beide Stämme bei pH 3 bei über 2 Stunden eine 100% Überlebensfähigkeit zeigen.
Zusätzlich vorteilhafte Eigenschaften sind, das LA-5 kann auch in niedrigen ph-Bereichen wachsen kann. Bb-12 hat eine symbiotische Fermentationseigenschaft mit LA-5; es handelt sich bei beiden Bakterienstämmen um lactatbildende Bakterien. Beide Stämme haben in Versuchen auch eine besonders gute Lagerstabilität gezeigt.
Die Lagerstabilität des in der Kapsel enthaltenen Bakterienlyophilisats im Sinne des oben gesagten ist niemals alleine von der erfindungsgemässen Dareichungsform, sondern stets auch vom verwandten Bakterienstamm abhängig. Als Alternative zur Verwendung von besonders magensaftresistenten Bakterien bietet sich eine zusätzliche Beschichtung einer erfindungsgemässen Kapsel, vorzugsweise aus HPMC-Material, mit einer magensaftresistenten Masse an. Geeignete Beschichtungen sind z.B. Schellack, HPMCAS oder ähnliche Polysaccharidüberzüge. Derartige Beschichtungen sind erst bei einem pH>5 in wässrigem Medium löslich.

In einer weiteren bevorzugten Ausführungsform besteht das Bakterienlyophilisat aus Bakterienstämmen, einzeln oder in Kombination, ausgewählt aus der Gruppe umfassend Lactobacillus acidophilus, Lactobacillus crispatus, Lactobacillus helveticus, Lactobacillus iansoni, Bifidobacterium bifidus, Bifidobacterium lactis, Bifidobacterium brevis, Bifidobacterium longum, Streptococcus thermophilus und Enterococcus faecis. Bei diesen Bakterienstämmen handelt es sich um die bevorzugt eingesetzten probiotischen Bakterienstämme, die bereits genannten Vorteil einer intakten, gutartigen Darmflora bewirken. Bevorzugt handelt es sich um Milchsäurebakterien, die durch ihre Ausscheidung mit Lactat einen niedrigen pH-Wert im Milieu der Darmflora erzeugen und so dem Auftreten pathogener Keime vorbeugt.

Eine weitere bevorzugte Ausführungsform ist eine erfindungsgemässe Kapsel eingeschlossen in eine wasserdampfsperrende Verpackung. Die wasserdampfsperrende Verpackung verhindert, dass bei erhöhter Luftfeuchtigkeit der geringe Wassergehalt von Füllmaterial und Kapselhülle durch Zutritt von Luftfeuchtigkeit während der Lagerung erhöht wird. Die wasserdampfsperrende Verpackung ermöglicht eine ausgedehnte Lagerung der erfindungsgemässen oralen Darreichungsform ohne (wesentliche) Veränderung des Wassergehalts. Eine Wasserdampfsperrende Verpackung im Sinne der vorliegenden Erfindung sind vor allem Glasflaschen mit Aluminiumdeckel und Dichtungsmaterial, es kann sich aber auch um Polyethylenflaschen, Aluverbundsäckchen oder Blisterverpackungen aus Materialien mit hohen Wasserdampfsperrwerten, z.B. PVDC-Folien (Polyvinylchlorid beschichtet mit Polyvinylidenchlorid) oder COC-Folien (Polypropylen laminated Cyclo-olefine-Copolymer PP/COC/PP, z.B. Pentapharm COC 30/300/30 der Firma Klöckner Pentaplast) oder COC-Multilayer Film (z.B.Topas-COC der Firma Tecona GmbH) handeln. Eine derartige Verpackung kann bei einem Wassergehalt der Gesamtkapsel von weniger als 7% die Wasseraufnahme (Sorption) der Kapselhülle und -füllstoffe auf unter 7% (bei 75% rH, 25°C) begrenzen.

Ein weiterer Gegenstand der vorliegenden Erfindung ist eine Kapsel für probiotische Bakterien umfassend eine Kapselhülle, Füllstoffe und lyophilisierte Bakterien, wobei die Kapselhülle aus einem Zellulosederivat besteht. Eine bevorzugte Ausführungsform weist einen Wassergehalt im Sinne des oben gesagten von 2-7% (w/w) auf.

Ein weiterer Gegenstand der vorliegenden Erfindung ist das Verfahren zur Herstellung einer mit Füllstoffen, vorzugsweise mit das Bakterienwachstum fördernden Füllstoffen, und lyophilisierten Bakterien, vorzugsweise ungeschützten lyophilisierten Bakterien, befüllten Steckkapsel, umfassend das Trocknen der Füllstoffe auf weniger als 2% (w/w) Wassergehalt, das Mischen der lyophilisierten Bakterien mit den Füllstoffen, das Einfüllen der Mischung in Kapseln (und, optional, das Versiegeln der Kapseln in einer wasserdampfsperrende Verpackung), wobei die Luftfeuchtigkeit während des gesamten Verfahrens höchstens 30% rH, vorzugsweise weniger als 10% rH beträgt. Ein Bakterienlyophylisat im Sinne des erfindungsgemässen Verfahrens ist ungeschützt, weist also keine zusätzliche, schützende Umhüllung bzw. Verkapselung z.B. durch eine getrocknete Gelmatrix auf.
In einer bevorzugten Ausführungsform des Verfahrens beträgt die Raumtemperatur und die Temperatur aller mit dem Bakterienlyophilisat in Berührung kommenden Teile höchstens 30°C. Dies vermindert zusätzlich thermischen, die Lebensfähigkeit der Bakterien beinträchtigenden, Stress und unerwünschte chemische Reaktionen. Beispielsweise können Amadori-Produkte durch Umsetzung von Zuckern mit Proteinen auch im Trockenzustand, vorzugsweise beim Erwärmen, entstehen.

### Beispiel 1

### Kapselherstellung

Maltodextrin, Maisstärke, Fructooligosaccharide (Matrix I) werden mit Trockenluft von <10% relativer Luftfeuchtigkeit (rH) bei 60°C während 2h (Wirbelschichtgranulator) oder 12 h (Hordenschrank) getrocknet und im geschlossenen Behälter (oder bei < 10% rH) auf ca. 20oC abgekühlt.
Gefriergetrocknete, lebende Bakterien werden im Röhnrad (Scherkraftmischer, keine Verdichtung und mechanische Belastung) in geschlossenem System mit der getrockneten Matrix I während 15 min. gemischt. Magensiumstearat wird während 2 min. untergemischt.
Die Steckkapseln werden während 24h bei 20-25°C und <30% rH konditioniert.
Die Mischung wird in klimatisierten Räumen (20-25°C und <30% rH) auf konventionellen Kapselfüllmaschinen in die Steckkapseln gefüllt und direkt anschliessend in die Endverpackung (Gläser oder COC-Blister) verpackt, oder in versiegelten Aluminium/Polyethylen-Verbundsäcken in bulk zwischengelagert.

| | |
|---|---|
| Lactobacillus acidophilus La-5 (10¹⁰CFU/g) | 20 mg |
| Bifidobakterium bifidum Bb-12 (10¹⁰CFU/g) | 20 mg |
| Maltodextrin | 200 mg |
| Maisstärke | 28 mg |
| Fructo-oligosaccharide | 114 mg |
| Magnesiumstearat | 38 mg |
| Kapselhülle (HPMC) VEGICAPs™* Grösse 0 | 95 mg (0.68 ml Volumen) |

| | |
|---|---|
| * (Hersteller: GS Technologies Inc./Iowa) | |

### Beispiel 2

### Kapselherstellung

Herstellung wie in Bsp. 1, jedoch mit folgeder Zusammensetzung:

| | |
|---|---|
| Lactobacillus acidophilus La-5 (10¹⁰CFU/g) | 200 mg |
| Maltodextrin | 115 mg |
| Maisstärke | 15 mg |
| Fructo-oligosaccharide | 65 mg |
| Magnesiumstearat | 20 mg |
| Kapselhülle (HPMC) VEGICAPS™ Grösse 0 | 95 mg (0.68 ml Volumen) |
| * (Hersteller: GS Technologies Inc./Iowa) | |

### Beispiel 3

### Überlebensrate nach Lagerung der Kapseln

Mit Bakterien der Stämme Lactobacillus La-5 (Chr. Hansen) bzw. Bifidobakterium Bb-12 (Chr. Hansen) und Füllstoffmischung gemäss Beispiel 2 hergestellte und in Gläser verpackte Kapseln wurden 0-8 Monate bei 25°C gelagert.Einzelne Kapseln wurden nach verschiedenen Zeiten entnommen und der Kapselinhalt in sterilem Phospatpuffer (6.25 mM, pH=7.2) einer Reihenverdünnung unterzogen. Parallel hergestellte Kapseln aus Gelatine dienen als Kontrolle. 1 ml jeder Verdünnung wurde auf sterilen Petrischalen ausgestrichen und bei 37°C drei Tage inkubiert.

### Medien:

- Lactobacillus:: MRS-IM Agar (Fa. Chr. Hansen) mit Sucrose und Brom-Cresol-Purple; aerobe Inkubation
- Bifidobakterium:: MRS-IM Agar (Fa. Chr. Hansen) mit Glucose und lmg/ml LiCl, 0.5 mg/ml Cystein-HCl, 0.5 mg/l Dichloxallin; ana erobe Inkubation

Die Kolonienzahl wurde dann durch Auszählen bestimmt als CFU (Colony forming units). Die Überlebensrate wird als Quotient aus CFU(t=3,6..)/CFU(t=0) für eine gegebene Verdünnung bestimmt.

| La-5 | | | | |
|---|---|---|---|---|
| Monate | CFU: Vegicaps (in 10⁸ bzw. % survival) | | CFU: Gelatinekapseln (in 10⁸ bzw. % survival) | |
| 0 | 4.2 | 100% | 3.2 | 100% |
| 3 | 4.0 | 96 | 2.8 | 88 |
| 6 | 3.4 | 81 | 2.4 | 74 |
| 8 | 3,0 | 71 | 1.6 | 50 |

| Bb-12 | | | | |
|---|---|---|---|---|
| Monate | CFU: Vegicaps (in 10⁸ bzw. % survival) | | CFU: Gelatinekapseln (in 10⁸ bzw. % survival) | |
| 0 | 2,0 | 100% | 5,0 | 100% |
| 3 | 2.2 | 110 | 2.4 | 48 |
| 6 | 2.1 | 105 | 1.9 | 38 |

## Patentansprüche

1. Eine Kapsel für Freisetzung von Bakterien, in einer Kapselhülle enthaltend mindestens ein Bakterienlyophilisat, vorzugsweise lyophilisierte Milchsäurebakterien, und mindestens einen Füllstoff, dadurch gekennzeichnet, dass der Wassergehalt der Kapselhülle höchstens 7% (w/w), vorzugsweise weniger als 3% (w/w) beträgt.

2. Eine Kapsel gemäss Anspruch 1, dadurch gekennzeichnet, dass die Kapselhülle aus einem Cellulosederivat, vorzugsweise Hydroxypropylmethyl-Cellulose (HPMC), besteht.

3. Eine Kapsel gemäss Anspruch 1, dadurch gekennzeichnet, dass die Kapselhülle aus Stärke oder einem Stärkederivat besteht.

4. Eine Kapsel gemäss Anspruch 1 bis 3, dadurch gekennzeichnet, dass die Füllstoffe zum der Kapselherstellung einen Wassergehalt von höchstens 2% (w/w), vorzugsweise von 1% (w/w) oder weniger haben.

5. Eine Kapsel gemäss Anspruch 1-4, dadurch gekennzeichnet, dass die Füllstoffe das Wachstum von Bakterien, vorzugsweise das Wachstum von Milchsäurebakterien, fördernde Substanzen umfasst.

6. Eine Kapsel gemäss Anspruch 5, dadurch gekennzeichnet, dass die Füllstoffe mindestens Lactose, Frukto-oligosaccharide oder eine Mischung daraus umfassen.

7. Eine Kapsel gemäss einem der vorhergehenden Ansprüche, dadurch gekennzeichnet, dass das Bakterienlyophilisat einen oder mehrere magensaftresistente, vorzugsweise gallentolerante Bakterienstämmen umfasst.

8. Eine Kapsel gemäss einem der vorhergehenden Ansprüche, dadurch gekennzeichnet, dass das Bakterienlyophilisat aus Bakterienstämmen besteht, einzeln oder in Kombination ausgewählt aus der Gruppe umfassend Lactobacillus acidophilus, Lactobacillus crispatus, Lactobacillus helveticus, Lactobacillus iansoni, Bifidobacterium bifidus, Bifidobacterium lactis, Bifidobacterium brevis, Bifidobacterium longum, Streptococcus thermophilus und Entrococcus faecis.

9. Kapsel gemäss einem der vorhergehenden Ansprüche, dadurch gekennzeichnet, das die Kapsel in einer wasserdampfsperrenden Verpackung eingeschlossen ist.

10. Kapsel für probiotische Bakterien, umfassend wenigstens eine Kapselhülle, einen Füllstoffe und ein Bakterienlyophilisat, dadurch gekennzeichnet, dass die Kapselhülle aus einem Cellulosederivat, vorzugsweise einem veretherten Cellulosederivat, besteht.

11. Kapsel gemäss Anspruch 10, dadurch gekennzeichnet, dass die Kapselhülle aus Hydroxypropylmethyl-Cellulose (HPMC), vorzugsweise aus HPMC mit einem Wassergehalt von 2-7% (w/w), besteht.

12. Verfahren zur Herstellung einer mit Füllstoffen, vorzugsweise mit das Bakterienwachstum fördernden Füllstoffen, und lyophilisierten Bakterien, vorzugsweise ungeschützten lyophilisierten Bakterien, befüllten Steckkapsel, umfassend
- Trocknen der Füllstoffe, auf weniger als 2% (w/w) Wassergehalt
- Mischen der lyophilisierten Bakterien mit den Füllstoffen
- Einfüllen der Mischung in Kapseln, wobei die relative Luftfeuchtigkeit während des gesamten Verfahrens höchstens 30% rH, vorzugsweise weni ger als 10% rH, beträgt.
- optional, Versiegeln der Kapseln in einer wasserdampfsperrenden Verpac kung

13. Verfahren gemäss Anspruch 12, dadurch gekennzeichnet, dass während des gesamten Herstellungsverfahrens die Lufttemperatur und die Temperatur aller mit dem Bakterienlyophilisat in Kontakt kommender Teile höchstens 30°C beträgt.
